# EUROPEAN PATENT SPECIFICATION

(11) **EP 0 938 467 B1**
(45) Date of publication and mention of the grant of the patent: **12.06.2002**
(21) Application number: 97946623.2
(22) Date of filing: 07.11.1997
(51) Int. Cl.: C07C 231/18, C07C 233/43, C07C 213/02, C07C 217/86, C07C 233/25, C07C 205/37

(54) **PROCESS FOR THE PREPARATION OF OPTICALLY PURE ISOMERS OF FORMOTEROL**
VERFAHREN ZUR HERSTELLUNG VON OPTISCH REINEN ISOMEREN DES FORMOTEROLS
PROCEDE DE PREPARATION D'ISOMERES DE FORMOTEROL OPTIQUEMENT PURS

(30) Priority: 12.11.1996 US 747592
(43) Date of publication of application: 01.09.1999
(73) Proprietor: SEPRACOR, INC., Marlborough, MA 01752 (US)
(72) Inventor: GAO, Yun, Southborough, MA 01772 (US); HETT, Robert, 5000 Aarau (CH); FANG, Kevin, Q., Wellesley, MA 02181 (US); WALD, Stephen, A., Sudbury, MA 01776 (US); SENANAYAKE, Chris, H., Shrewsbury, MA 01545 (US)
(74) Representative: Price, Vincent Andrew
(86) International application number: US9720472
(87) International publication number: WO9821175

(56) References cited:
- WO-A-92/05147
- ROBERT HETT ET AL.: "Enantio- and Diastereoselective Synthesis of all Four Stereoisomers of Formotero " TETRAHEDRON LETTERS, vol. 38, no. 7, February 1997, OXFORD GB, pages 1125-1128, XP002057058
- CHEMICAL ABSTRACTS, vol. 127, no. 12, 22 September 1997 Columbus, Ohio, US; abstract no. 161695m, SHIRO MIYOSHI ET AL.: "Preparation of tricyclic compounds as beta-3 adrenaline receptor antagonists." page 654; column 1; XP002057061 & WO 97 25311 A (ASAHI KASEI) 17 July 1997
- K. MURASE ET AL.: "Absolute Configurations of Four Isomers of 3-Formamido-4-hydroxy-alpha[[N-(p-methoxy- alpha-methylphenethyl)amino]methyl]benzyl Alcohol, a Potent beta-Adrenoreceptor Stimulant" CHEMICAL AND PHARMACEUTICAL BULLETIN, vol. 26, no. 4, April 1978, TOKYO JP, pages 1123-1125, XP002057059 cited in the application
- JAN TROFAST ET AL.: "Steric Aspects of Agonism and Antagonism at beta-Adrenoreceptors: Synthesis of and Pharmacological Experiments With the Enantiomers of Formoterol and Their Diastereomers " CHIRALITY, vol. 3, no. 6, 1991, pages 443-450, XP002057060 cited in the application

## Description

The present invention relates to a method of preparation of optically pure isomers of formoterol, especially the (*R,R*)- and (*S,S*)-isomer, by the reaction of an optically pure 4-benzyloxy-3-formamidostyrene oxide with an optically pure 4-methoxy-α-methyl-*N*-(phenylmethyl)benzeneethanamine followed by debenzylation.

### Background of the Invention

Formoterol, whose chemical name is (+/-) *N*-[2-hydroxy-5-[1-hydroxy-2[[2-(p-methoxyphenyl)-2-propyl]amino]ethyl]phenyl]-formamide, is a highly potent and β₂-selective adrenoceptor agonist having a long lasting bronchodilating effect when inhaled. The structure of formoterol is as shown:

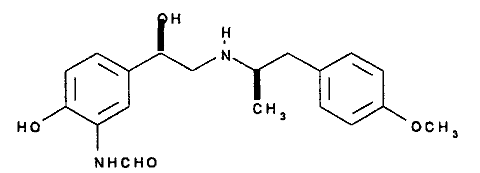

Formoterol has two chiral centers in the molecule, each of which can exist in two possible configurations. This gives rise to four combinations: (*R,R*), (*S,S*), (*R,S*) and (*S,R*). (*R,R*) and (*S,S*) are mirror images of each other and are therefore enantiomers; (*R,S*) and (*S,R*) are similarly an enantiomeric pair. The mirror images of (*R,R*) and (*S,S*) are not, however, superimposable on (*R,S*) and (*S,R*), which are diastereomers. Formoterol is available commercially only as a racemic diastereomer, (*R,R*) plus (*S,S*) in a 1:1 ratio, and the generic name formoterol refers to this enantiomeric mixture. The racemic mixture that is commercially available for administration is a dihydrate of the fumarate salt.

The graphic representations of racemic, ambiscalemic and scalemic or enantiomerically pure compounds used herein are taken from Maehr J. Chem. Ed. 62, 114-120 (1985): solid and broken wedges are used to denote the absolute configuration of a chiral element; wavy lines indicate disavowal of any stereochemical implication which the bond it represents could generate; solid and broken bold lines are geometric descriptors indicating the relative configuration shown but denoting racemic character; and wedge outlines and dotted or broken lines denote enantiomerically pure compounds of indeterminate absolute configuration. Thus, the formula for formoterol above reflects the racemic nature of the commercial material, while among the structures below, those having open wedges are intended to encompass both of the pure enantiomers of that pair and those having solid wedges are intended to encompass the single, pure enantiomer having the absolute stereochemistry shown.

All four isomers of formoterol have been synthesized and briefly examined for relaxing activity on the guinea pig trachea [Murase *et al.,* Chem, Pharm, Bull, 26, 1123-1129 (1978). It was found that the (*R,R*)-isomer is the most potent, while the others are 3-14 times less potent. More recently, the four isomers have been examined with respect to their ability to interact in vitro with β-adrenoceptors in tissues isolated from guinea pig [Trofast *et al.,* Chirality 3, 443-450 (1991)]. The order of potency was (*R,R*) >> (*R,S*)= (*S,R*) > (*S,S*). It was found that the (*R,R*)-isomer is 1000-fold more potent than the (*S,S*)-isomer. Preliminary research indicates that administration of the pure (R,R)-isomer may offer an improved therapeutic ratio.

Two reports have been published describing the synthesis of all four isomers of formoterol. In the first report [Murase *et al. op. cit*.], the (*R,R*)- and (*S,S*)- isomers were obtained by diastereomeric crystallization of racemic formoterol with tartaric acid. In the second report [Trofast *et al. op. cit*.], racemic 4-benzyloxy-3-nitrostyrene oxide was coupled with an optically pure (*R,R*)- or (*S,S*)-*N*-(1-phenylethyl)-*N*-(1-(p-methoxyphenyl)-2-propyl)amine to give a diastereomeric mixture of formoterol precursors, which were then separated by semipreparative HPLC and transformed to the pure formoterol isomers. Both syntheses suffer long synthetic procedure and low overall yield and are impractical for large scale production of optically pure (*R,R*)- or (*S,S*)-formoterol. For example, the Trofast reference describes reacting 4.5 grams of the styrene oxide with 4.8 grams of the phenethylamine to produce 94 milligrams of the pure *S,S* enantiomer. Therefore, there exists a need for a more economical and efficient method of making optically pure formoterol.

### Summary of the Invention

The processes of the invention provide a practical synthesis of optically pure formoterol, especially (*R,R*)- and (*S,S*)-formoterol.

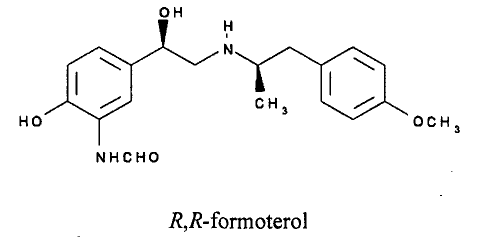

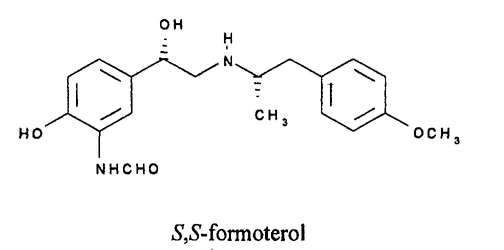

In its broadest aspect, the invention relates to a process for preparing a compound of formula F 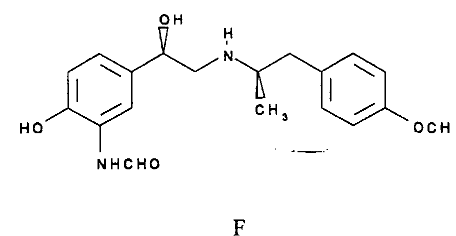 or a salt thereof, comprising the sequential steps of: (a) reacting a compound of formula 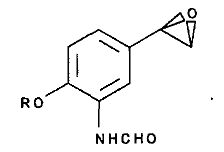 wherein R is benzyl or substituted benzyl,
with a compound of formula FBA: 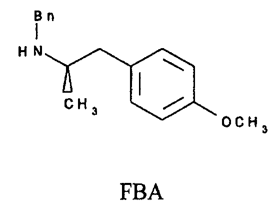 and (b) reducing with hydrogen gas in the presence of a noble metal catalyst.

The term "substituted benzyl" refers to any protecting group for a phenol that contains the benzyl (or phenylmethyl) nucleus substituted with one or more substituents that do not interfere with its function as a protecting group. Suitable substituents include: C₁ to C₆-alkyl, C₁ to C₆-alkoxyl, halogen and combinations thereof. In a particular embodiment, R is benzyl (Bn), and the compound is referred to herein as FAE:

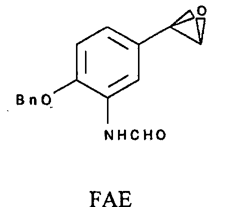

The epoxide may be produced *in situ* from the corresponding bromohydrin: 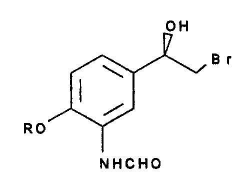 by treatment with a base, and the benzylamine may be produced *in situ* from a corresponding salt by treatment with a base. In one embodiment, the steps may be combined to provide a process wherein a compound of formula FBH3: 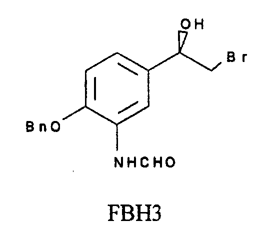 a compound of formula FBA-HA: 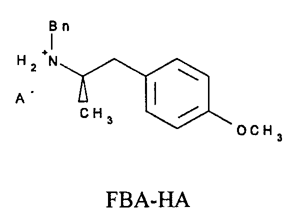 and at least one equivalent of a base are combined to produce a mixture comprising an epoxide and a free base. The mixture of epoxide and free base is heated at a temperature sufficient to cause a reaction to produce a benzyl-protected aminoalcohol, and the benzyl-protected aminoalcohol is reduced with a source of hydrogen in the presence of a noble metal catalyst. In the above structure A⁻ is the anion of a conjugate acid HA having a pKa sufficient to protonate the amine.

In the foregoing processes a preferred noble metal catalyst is palladium and a preferred base is an alkali metal carbonate, particularly potassium carbonate. The source of hydrogen may be hydrogen gas or a hydrogen-donating compound such as ammonium formate.

Suitable acid addition salts for the compounds of the present invention include for example, acetic, benzenesulfonic (besylate), benzoic, camphorsulfonic, citric, ethenesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric acid, p-toluenesulfonic, and the like. The mandelic acid salt is especially preferred for compounds of formula FBA; the tartrate and fumarate are preferred for formoterol enantiomers F.

In another aspect, the invention relates to a process for synthesizing a compound of formula 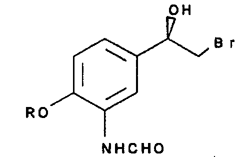 comprising the sequential steps of (a) reducing 2-bromo-4'-RO-3'-nitroacetophenone with about one equivalent of borane-methyl sulfide in the presence of a catalytic amount of a single enantiomer of an oxazaborolidine reagent derived from a chiral aminoalcohol, preferably from *cis* 1-amino-2-indanol, to produce substantially enantiomerically pure α-(bromomethyl)-4-RO-3-nitrobenzenemethanol: 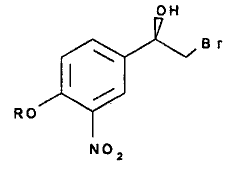 (b) reducing the α-(bromomethyl)-4-RO-3-nitrobenzenemethanol with hydrogen in the presence of a noble metal catalyst to produce an aniline; and (c) formylating the aniline with formic acid and acetic anhydride. A preferred noble metal catalyst for this process is platinum, derived from PtO₂. Steps (b) and (c) may be carried out without isolation of the aniline. In a preferred embodiment, R is benzyl and 2-bromo-4'-benzyloxy-3'-nitroacetophenone is reduced to produce substantially enantiomerically pure α-(bromomethyl)-4-phenylmethoxy-3-nitrobenzenemethanol (FBH):

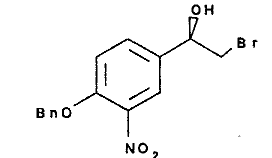

In a more preferred embodiment the single enantiomer of an oxazaborolidine is derived from (1*R*,2*S*)-1-amino-2-indanol, which produces α-(bromomethyl)-4-phenylmethoxy-3-nitrobenzenemethanol of the *R* configuration. The oxazaborolidine may be generated *in situ* from (1R,2S)-1-amino-2-indanol and two equivalents of borane-methyl sulfide.

In another aspect, the invention relates to a process for preparing a substantially enantiomerically pure salt of 4-methoxy-α-methyl-*N*-(phenylmethyl)benzeneethanamine of formula FBA-HA 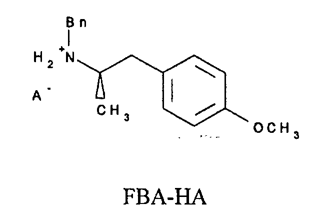 comprising: (a) reducing 4-methoxyphenyl acetone with hydrogen in the presence of a platinum catalyst and about 1 equivalent of benzylamine in methanol; (b) adding about one equivalent of a single enantiomer of mandelic acid; (c) heating to obtain a methanolic solution; (d) cooling to obtain a crystalline solid phase; and (e) recovering the crystalline solid from the methanolic solution. If desired, one may convert the crystalline mandelic acid salt from step (e) to a salt of an acid other than mandelic acid by processes well known in the art.

In another aspect, the invention relates to an overall process for preparing a compound of formula F: 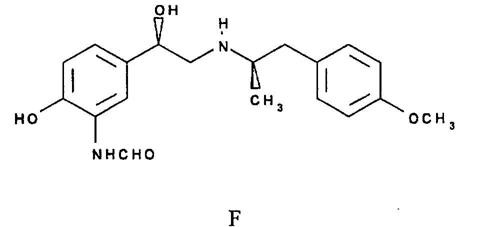 from 2-bromo-4'-benzyloxy-3'-nitroacetophenone and 4-methoxy-α-methyl-N-(phenylmethyl)benzeneethanamine comprising the sequential steps of: (a) reducing 2-bromo-4'-benzyloxy-3'-nitroacetophenone with about one equivalent of borane-methyl sulfide in the presence of a catalytic amount of a single enantiomer of an oxazaborolidine derived from *cis* 1-amino-2-indanol to produce substantially enantiomerically pure α-(bromomethyl)-4-phenylmethoxy-3-nitrobenzenemethanol (FBH); (b) reducing the α-(bromomethyl)-4-phenylmethoxy-3-nitrobenzenemethanol with hydrogen in the presence of a noble metal catalyst to produce an aniline FBH2; (c) formylating the aniline with formic acid and acetic anhydride to produce a compound of formula FBH3: 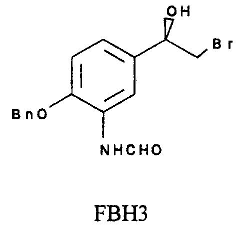 (d) combining FBH3, a salt of 4-methoxy-α-methyl-*N*-(phenylmethyl)benzeneethanamine (FBA-HA) and at least one equivalent of a base to produce a mixture comprising an epoxide (FAE) and a free base (FBA); (e) heating the mixture of epoxide and free base at a temperature sufficient to cause a reaction to produce a benzyl-protected aminoalcohol (DBF); and (f) reducing the benzyl-protected aminoalcohol with hydrogen gas in the presence of a noble metal catalyst.

In another aspect, the invention relates to compounds of formula: 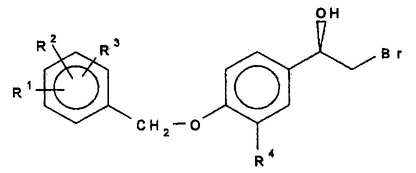 wherein each of R¹, R² and R³ is independently chosen from the group consisting of hydrogen, C₁ to C₆-alkyl, C₁ to C₆-alkoxyl, and halogen and R⁴ is -NO₂, -NH₂ or -NHCHO. The compounds are useful as intermediates in the synthesis of single enantiomers of formoterol. The compounds in which all of R¹, R² and R³ are hydrogen are preferred.

In another aspect, the invention relates to the *L*-(+)-tartrate salt of *R,R*-formoterol, which is unexpectedly superior to other salts of *R,R*-formoterol in that it is easy to handle, pharmaceutically innocuous and non-hygroscopic. The *D*-(-)-tartrate salt of *S,S*-formoterol possesses similar advantages.

### Detailed Description

The present invention relates to a more practical and efficient process for the preparation of optically pure isomers of formoterol. This method is particularly advantageous in comparison with known methods because it utilizes optically pure precursors that are readily available by simple resolution and asymmetric reduction. The overall sequence is set forth in Scheme 1, wherein R has been exemplified as benzyl. The same sequence could be used to produce other intermediates in which R is substituted benzyl by beginning with the appropriate starting material analogous to FBK. Brackets indicate intermediates that could be isolated but are not usually isolated in the integrated process.

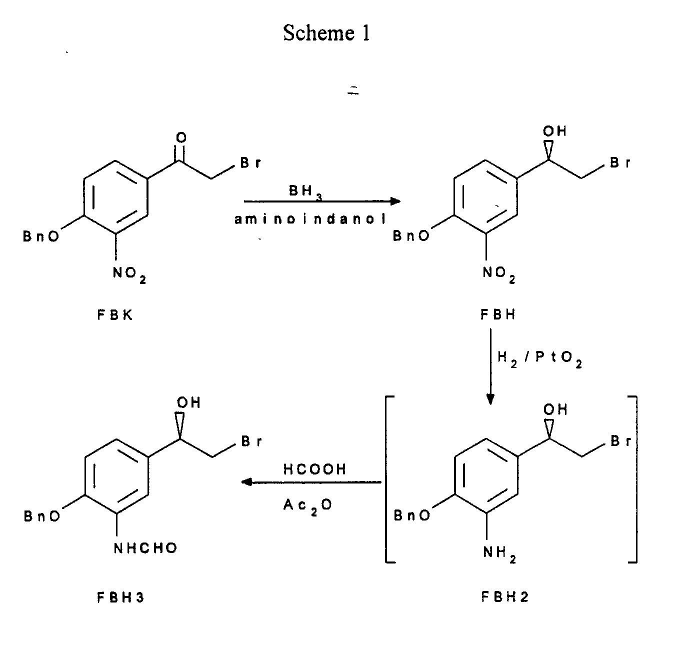

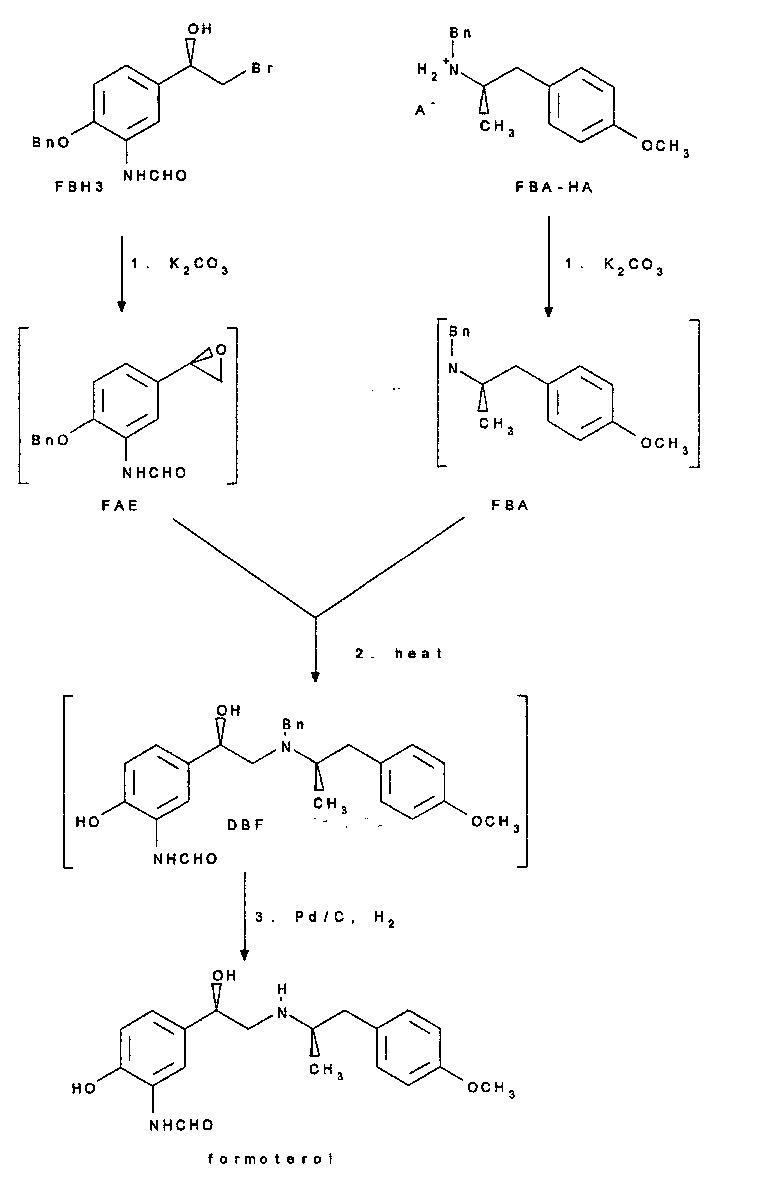

In the process described above, the optically pure 4-methoxy-α-methyl-*N*-(phenylmethyl)benzene-ethanamine, also called 2-*N*-benzylamino-1-(p-methoxyphenyl)propane (FBA), is obtained by resolution of the racemic compound with *L*- or (*D*)-mandelic acid using a modification of the procedure of Kraft, *et al.* [Rec. Trav. Chim, Pays-Bas 85, 607 (1966)]. The racemic *N*-benzylamine compound was prepared by the reductive amination of p-methoxyphenylacetone with *N*-benzylamine under catalytic hydrogenation, but other reductive conditions using methods known in the art could be used. (*See,* Houben-Weyl's Methoden der Org. Chem. Band IV/1c, p427.)

The invention encompasses a process for making optically pure formoterol from optically pure 4-benzyloxy-3-formamidostyrene oxide (FAE) comprising the coupling and hydrogenation described above in combination with a method for the preparation of the optically pure styrene oxides. According to this aspect the optically pure styrene oxide is obtained by: (a) reduction of 2'-bromo-4-benzyloxy-3-nitroacetophenone with borane stereoselectively in the presence of a chiral oxazaborolidine catalyst to give the corresponding optically active bromohydrin [*See,* Hong, *et al.,* Tetrahedron Lett, 35, 6631(1994)] and U.S. Patent 5,495,821]; (b) reduction of the 3-nitro group in the bromohydrin with a reducing agent to the amino group followed by formylation with formic acid or formic acid/acetic anhydride (Ac₂O) to give the 3-formamido bromohydrin FBH3; and (c) conversion of the 3-formamido bromohydrin to the corresponding 4-benzyloxy-3-formamidostyrene oxide FAE with a base.

The optically pure 2-*N*-benzylamino-1-(p-methoxyphenyl)propane (FBA) is obtained by resolution of the racemic compound with *L*- or (*D*)-mandelic acid. The resolution of racemic *N*-benzylamine compound is performed using one equivalent of *L*-or *D*-mandelic acid in an alcohol solvent such as methanol (MeOH). Optically pure benzylamine mandelic acid salt (FBA-MA) is obtained after four or five crystallizations. The free *N*-benzylamine compound is then obtained by treating the mandelic acid salt with a base such as aq. NaOH or aq. Na₂CO₃ or aq. NH₃ in the presence of an inert organic solvent such as *t*-butyl methyl ether (MTBE) or ethyl acetate (EtOAc) followed by evaporation of the solvent. (*R*)-2-*N*-benzylamino-1-(p-methoxyphenyl)propane is obtained from the *L*-(+)-mandelic acid salt while the (*S*)-enantiomer is obtained from the *D*-(-)-mandelic acid salt. From the same lot of racemic *N*-benzylamine compound, both (*R*)- and (*S*)-enantiomer can be obtained by using the appropriate mandelic acid.

The optically pure epoxide (FAE) is prepared from commercially available 4-benzyloxy-3-nitroacetophenone. Thus, the acetophenone may be bromonated with bromine in an inert organic solvent such as CH₃CN, MeOH or chloroform to give the α-bromoacetophenone. The bromoacetophenone is then reduced with a borane reducing agent such as BH₃·THF or BH₃·Me₂S in the presence of a chiral oxazaborolidine catalyst such as *cis*-(1*R*,2*S*)-aminoindanol-B-Me catalyst to give the optically active bromohydrin after isolation by crystallization in >96% ee. The bromohydrin can be further enriched to >98% ee by recrystallization. The absolute configuration of the bromohydrin is determined by the chirality of the oxazaborolidine catalyst. The nitro group in the bromohydrin is selectively reduced to the amine group using a reducing agent known for selective nitro reduction, such as Sn, Fe with acid, SnCl₂ or by heterogeneous catalytic hydrogenation in the presence of a noble metal catalyst such as PtO₂ or Pt/C. The amine group is then formylated with a mixture of formic acid and acetic anhydride without racemization, and the resulting compound is converted to optically pure 4-benzyloxy-3-formamidostyrene oxide with a base such as aq. NaOH or K₂CO₃ in an alcohol solvent or solvent mixture such as MeOH/THF. The epoxide obtained can be purified by recrystallization from an inert organic solvent or solvent mixture, preferably from EtOAc/heptane or toluene/heptane.

The optically pure 2-*N*-benzylamino-1-(*p*-methoxyphenyl) propane (FBA) is reacted with optically pure 4-benzyloxy-1-formamidostyrene oxide without racemization to give an optically pure N,O-di-benzylformoterol intermediate (DBF), and the N,O-di benzyl group of the dibenzylformoterol is removed by hydrogenation in the presence of a hydrogenation catalyst, to give optically pure formoterol. Alternatively, the dibenzylformoterol is obtained directly from the reaction of optically pure 2-*N*-benzylamino-1-(p-methoxyphenyl)propane with the optically pure 1-(4'-benzyloxy-3'-formamidophenyl)-2-bromoethanol (FBH3) in the presence of a base whereby the epoxide (FAE) is formed *in situ.*

For the synthesis of optically pure formoterol, the optically pure *N*-benzylamine sidechain may be coupled with the epoxide without solvent at temperature in the range of 100-140° C, or in a high boiling inert solvent under reflux. Suitable solvents include toluene, *t*-butanol, *t*-amylalcohol, and methyl isobutylketone (MIBK). The resulting dibenzylformoterol (DBF) can be purified by column chromatography or by recrystallization as salt of an organic acid such as fumaric acid. It can also be used directly without purification for the de-benzylation reaction to form formoterol.

The dibenzylformoterol product is converted by catalytic hydrogenation in the presence of Pd catalyst such as Pd/C directly to optically pure formoterol. This reaction is preferably performed in an alcohol solvent such as methanol, ethanol, or 2-propanol at 40-60 psi of hydrogen pressure and 15-30°C for 2-15 hours. Although the formoterol product can be isolated as the fumaric acid salt by adding fumaric acid to the reaction solution after removal of the catalyst, a product of higher purity is obtained if the formoterol is recovered and purified as the tartrate salt and then converted to the fumarate. Alternatively, the hydrogenation (de-benzylation) can be performed in the presence of the appropriate organic acid in an alcohol solvent such as MeOH under similar conditions. The resulting formoterol acid salt is then isolated by crystallization by addition of a less polar co-solvent after filtration to remove the catalyst.

In a specific synthesis, the enantioselective reduction of FBK was done with 15 mol% AIBMe catalyst at -15°C. The bromohydrin was isolated after aqueous work-up with enantioselectivities ranging from 96-98% isomeric purity. The catalyst AIBMe was generated from aminoindanol and trimethylboroxine, followed by azeotropic removal of by-products using toluene. When the FBH was not purified by crystallization, 2-4% of minor isomer was carried through the synthetic sequence and caused lower yields in the last step. In those cases it was necessary to crystallize with *L*-tartaric acid 3-4 times at the last step in order to obtain the desired enantiomeric purity (>99.5%) of formoterol tartaric acid salt.

The chiral amine, 4-methoxy-α-methyl-*N*-(phenylmethyl)benzeneethanamine, was synthesized by a reductive amination procedure followed by a novel resolution procedure with mandelic acid. A concentration of 0.4M appears to be the optimal concentration and provides the product after 3-4 crystallizations in isomeric purities of 99.5-100%.

In Scheme 1, the aniline (FBH2) can be isolated as an intermediate and then transformed to the epoxide, but FBH2 has a tendency to oxidize when exposed to air. Therefore, there is an advantage to not isolating the FBH2 and instead hydrogenating in THF, which allows the formylation directly after filtration of the catalyst. The formamidobromohydrin (FBH3), as a highly crystalline compound, can be isolated from the reaction mixture without aqueous work-up. Using pure FBH3 and forming the epoxide provides crystalline FAE.

The epoxide opening reaction was conducted as neat reaction with the free amine to give the penultimate precursor dibenzylformoterol (DBF), as an oil with a purity of 85-87%. The reaction may also be run in toluene, 2-propanol or t-amyl alcohol.

Crude DBF can be converted to formoterol tartrate, which can be crystallized in high yields and high purities, and formoterol fumarate can be generated by salt switch from the purified formoterol tartrate. Although formoterol fumarate can also be crystallized directly from the hydrogenation mixture in high yields, subsequent crystallizations do not remove a major impurity.

In the enantioselective reduction of FBK to FBH, an AIBMe catalyst consistently gives slightly higher selectivities than the AIBH, but it is more difficult to prepare, more expensive and the optimum process temperature is lower than that of the AIBH process.

Epoxide formation from FBH3 and release of the free base from the benzylamine FBA-HA may be accomplished in separate steps. However, since both reactions require a base, a combination of both steps into a one pot procedure is possible and simplifies the process.

### Experimental

### 2-Bromo-4'-benzyloxy-3'-nitroacetophenone (FBK)

A 5-liter flask was charged with 300 g (1.1 mol) of 4-benzyloxy-3-nitroacetophenone and 3 liters of acetonitrile. The mixture was heated to 50° C to form a clear solution, and 180 g of bromine (1.6 mol) was added in one portion. The reaction was stirred at 50° for 15-25 minutes, during which time the deep red color changed to pale orange and TLC (ethyl acetate/hexane 3:7) showed no remaining starting material. Without heating, 200 to 300 mL of acetonitrile, along with the byproduct hydrogen bromide, were distilled from the reaction under vacuum. During the course of the distillation, the temperature dropped to about 15° and the product precipitated as a yellow solid. The reaction was stirred at 0-5° for two hours and the product filtered off and washed with acetonitrile. The resulting 2-bromo 4'-benzyloxy-3'nitroacetophenone was dried in vacuum to yield 242 g (63%) of off-white solid having a melting point of 136°C.

### R-α-(bromomethyl)-4-phenylmethoxy-3-nitrobenzemethanol (FBH)

A 2-liter flask was charged with 2.5 g (17 mmol) of (1*R*,2*S*)-aminoindanol in 50 mL of THF under argon. While cooling to maintain a temperature below 25° C, 3.4 mL (34 mmol) of a 10 mol solution of borane methyl sulfide was added over a period of 5 minutes and the reaction stirred for ten minutes at 25° C to complete formation of the catalyst. To this catalyst solution the ketone and reducing agent were added simultaneously. From separate reservoirs were added (1) a solution of 120 g of FBK (0.34 mol) in 950 mL of THF and (2) 24 mL of 10 M borane-methyl sulfide. Addition was over a period of 3 hours at 25° C. The reaction was cooled on an ice bath and 100 mL of methanol was added over a period of 15 minutes. The reaction mixture was concentrated under vacuum to a volume of about 200 mL, and 650 mL of toluene was added to dissolve the residue. The solution was washed with 0.2 M sulfuric acid and then water. If desired the aminoindanol may be recovered from the aqueous acidic phase. The organic phase was dried over sodium sulfate, filtered and concentrated to a weight of 240-260 g. A total of 100 mL of heptane was added to the mixture with stirring at 50-60°, then cooled to 15-20° and filtered. Although the wet filter cake may be used in the next step without drying, the solid was dried under vacuum to give 95-108 g of (*R*)-FBH as an off white solid, melting point 68° C.

### N-[5-(2-bromo-1-hydroxyethyl)-2-(phenylmethoxy)phenyl]formamide (FBH3)

A solution of 100 g (0.28 mol) of (*R*) FBH in 200 mL of THF and 200 mL of toluene was hydrogenated in a Parr hydrogenator in the presence of 1 g of platinum oxide catalyst at 45-50 psi for 7-13 hours until hydrogen uptake ceased. The reaction mixture was filtered through a bed of diatomaceous earth and a solution of 21.5 g (0.48 mol) of formic aeid and 33 g (0.32 mol) of acetic anhydride, which had been pre-mixed, was added to the filtrate, which was maintained at 10-15° C by external cooling. The solution was stirred for 20 minutes at 10-25° C and then concentrated to about 300 mL at 30° C. One hundred milliliters of toluene was added and the reaction was stirred at 15° C for 15 minutes. The resulting slurry was filtered to provide 75 g (76% yield of (*R*)-FBH3 melting point 130°C, isomeric purity 99-99.5%. The product is also sometimes referred to as 2-bromo-(4'-benzyloxy-3'-formamidophenyl)ethanol.

### N- [5-oxiranyl-2-(phenylmethoxy)phenyl]formam ide (FAE)

If it is desired to isolate the epoxide, as opposed to generating it in situ in the next step, the following procedure may be used: a solution of 28 g of the aniline FBH2 from platinum catalyzed reduction of the nitro compound FBH was treated with a mixture of 17 mL of the mixed formic/acetic·anhydride, concentrated to dryness and dissolved in 200 mL of methanol. The methanolic solution was treated with 60 g of potassium carbonate, stirred at 30 minutes and concentrated under vacuum. The resulting residue was triturated with 400 mL of ethyl acetate, washed with water and brine, decolorized with carbon and dried over sodium sulfate. The drying agent and carbon were filtered off and the filtrate concentrated to give 19.3 g (86% yield) of the epoxide FAE as an oil, which solidified on standing (95.4% ee; m.p. 64-65° C).

### (R,R)-Formoterol-L-tartrate

A 2-liter flask was charged with 70 g of (*R*)FBH3 (0.2 mol) 76.5 g of (*R*)-FBA-*L*-MA (0.19 mol), 70 g of potassium carbonate (0.5 mol), 400 mL of THF and 400 mL of methanol. The mixture was stirred at 25° for 1-2 hours and the reaction followed by HPLC. When the starting material (FBH3) content was below 2%, the mixture was concentrated to dryness at 30-35 ° C under vacuum. To the residue were added in order, first 600 mL of toluene and then 600 mL of water. The slurry was stirred 10 minutes, the phases were separated and the organic phase was dried over sodium sulfate. The toluene solution was filtered free of drying agent and concentrated to 110 g. The residue, which was shown by HPLC to be a 1:1 mixture of FAE and FBA, was stirred under argon atmosphere at 110-130° C for 24 hours. To the hot mixture was added 400 mL of ethanol to obtain a clear solution of (*R,R*)DBF. The solution was cooled to 25°, transferred to a Parr hydrogenator and hydrogenated at 45-50 psi in the presence of 10 g of 5% palladium on carbon until hydrogen uptake was complete (3-4 hours). The mixture was filtered through a pad of diatomaceous earth washed with 200 mL of 2-propanol, and 28.5 g of *L*-tartaric acid (0.19 mol) and 60 mL of water was added to the filtrate. The mixture was heated to 60-80° C until a clear solution was formed. As soon as the clear solution formed, heating was discontinued and the mixture was cooled to 25°, at which temperature it was held for 1-2 hours. It was then further cooled to 0-5° for 1 hour and the product collected by filtration. The product was dried under vacuum to provide 70-80 g of (*R,R*) formoterol *L*-tartrate as an off white powder. The tartrate salt was dissolved in 700-800 mL of hot 80% aqueous 2-propanol, cooled as before and filtered again. The second recrystallization provided 60-70 g of (*R,R*) formoterol *L*-tartrate as an off-white powder having a melting point between 179 and 184 depending upon purity. A product having a chemical purity of 99.8% and an enantiomeric purity of 99.7% exhibits a melting point of 184° C.

### (R,R) Formoterol Fumarate

A 2-liter flask was charged with 650 mL of water and 60 g *of* (*R,R*) formoterol *L*-tartrate (0.12 mol). The mixture was stirred and 52 g of sodium bicarbonate (0.6 mol) was added in small portions. The product was extracted into 250 mL of ethyl acetate, dried over sodium sulfate, filtered and concentrated to give 56 g of the free base. The free base was dissolved in 260 mL of isopropyl alcohol and 7.0 g of fumaric acid (60 mmol) was added followed by 130 mL of 2-propanol. The mixture was heated to 50-60° until a clear solution was formed and then cooled as above to crystallize the fumarate salt. The product was filtered and washed with 2-propanol to provide 44 g of (*R,R*) formoterol fumarate as white crystals having a chemical purity greater than 98% and an enantiomeric purity greater than 99.5%.

### 4-Methoxy-α-methyl-N-(phenlymethyl)benzene ethanamine L-mandelic acid salt (FBA-L-MA)

To 800 mL of methanol were added 328 g of 4-methoxy-phenylacetone (2 mol) and 214 g of *N*-benzylamine (2 mol). The imine formation was exothermic and the solution warmed to 45° C. After reaction was complete, the solution was hydrogenated at 50 psi for 6-8 hours in the presence of 3.3 g of 5% platinum on carbon catalyst. When the hydrogen uptake had stopped, the reaction was filtered through diatomaceous earth, and the filter cake was washed with 200 mL of methanol. The combined filtrates were placed in a 6-liter flask and diluted with 4.2 liters of methanol. Three hundred four grams of (*S*)-*L*-mandelic acid (2 mol) was added and the mixture heated with stirring to reflux to obtain a clear solution. The solution was cooled to room temperature, stirred at room temperature for two hours and the mandelic acid salt filtered off. The recrystallization was repeated three times to obtain 60-70 g of (*R*)-FBA-*L*-MA having isomeric purity greater than 99.8% and a melting point of 164°C.

## Claims

1. A process for preparing a compound of formula 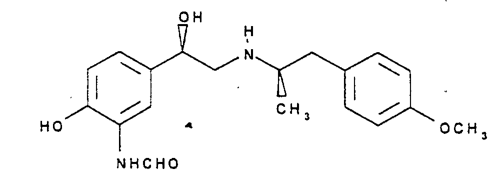 or a salt thereof, comprising the sequential steps of:
(a) reacting a compound of formula 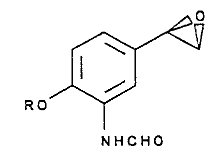 with a compound of formula 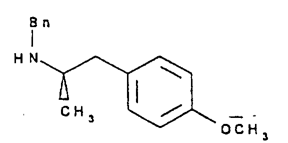 wherein R is benzyl or substituted benzyl, and
(b) reducing with a source of hydrogen in the presence of a noble metal catalyst.

2. A process according to claim 1 wherein said compound of formula 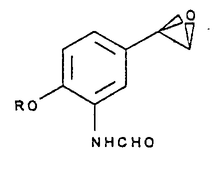 R = benzyl or substituted benzyl is produced *in situ* from the corresponding bromohydrin 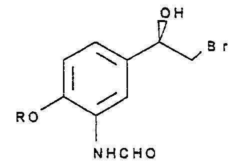 by treatment with a base, and/or wherein said compound of formula 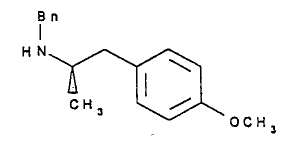 Bn = benzyl is produced *in situ* from a corresponding salt by treatment with a base.

3. A process according to claim 1 or claim 2 wherein said compound of formula 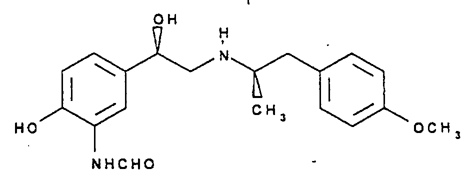 is : (a) of the *R,R* configuration, or (b) of the *S,S* configuration.

4. A process for preparing a compound of formula 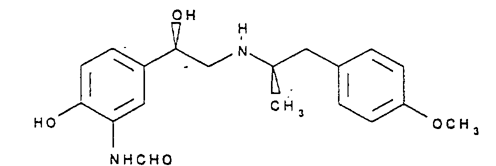 or salt thereof, comprising the sequential steps of:
(a) combining a compound of formula 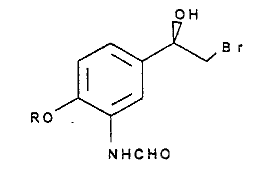 a compound of formula 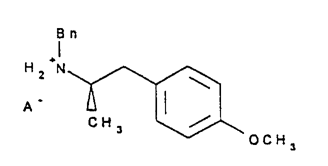 wherein R is benzyl or substituted benzyl and A⁻ is an acid counter ion, and at least one equivalent of a base to produce a mixture comprising an epoxide and a free base;
(b) heating said mixture of an epoxide and a free base at a temperature sufficient to cause a reaction to produce a benzyl-protected aminoalcohol; and
(c) reducing said benzyl-protected aminoalcohol with hydrogen gas in the presence of a noble metal catalyst.

5. A process according to any one of the preceding claims wherein said noble metal catalyst is palladium.

6. A process according to claim 2, or 4 wherein said base is an alkali metal carbonate.

7. A process according to any of claims 1 to 4 wherein said salt is a tartrate salt.

8. A process according to any of claims 1 to 4 wherein said salt is a fumarate salt.

9. A process for synthesizing a compound of formula 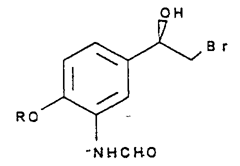 wherein R is benzyl or substituted benzyl, comprising the sequential steps of:
(a) reducing 2-bromo-4'-RO-3'-nitroacetophenone with about one equivalent of borane-methyl sulfide in the presence of a catalytic amount of a single enantiomer of an oxazaborolidine reagent to produce substantially enantiomerically pure α-(bromomethyl)-4-RO-3-nitrobenzenemethanol
(b) reducing said α-(bromomethyl)-4-RO-3-nitrobenzenemethanol with hydrogen in the presence of a noble metal catalyst to produce an aniline; and
(c) formylating said aniline with formic acid and acetic anhydride.

10. A process according to claim 9 wherein : (a) said oxazaborolidine reagent is generated *in situ* from (1R,2S)-1-amino-2-indanol and two equivalents of borane-methyl sulfide; and/or (b) said noble metal catalyst is platinum and said oxazaborolidine is derived from *cis* 1-amino-2-indanol; and/or (c) steps (b) and (c) are carried out without isolation of said aniline; and/or (d) said single enantiomer of an oxazaborolidine is derived from (1*R*,2*S*)-1-amino-2-indanol, and said substantially enantiomerically pure α-(bromomethyl)-4-RO-3-nitrobenzenemethanol is *R*-α-(bromomethyl)-4-phenylmethoxy-3-nitrobenzenemethanol.

11. A process for preparing a substantially enantiomerically pure salt of 4-methoxy-α-metnyl-*N*-(phenylmethyl)benzeneethanamine comprising:
(a) reducing 4-methoxyphenyl acetone with hydrogen in the presence of a platinum catalyst and 1 equivalent of benzylamine in methanol;
(b) adding one equivalent of a single enantiomer of mandelic acid;
(c) heating to obtain a methanolic solution;
(d) cooling to obtain a crystalline solid phase; and
(e) recovering said crystalline solid from said methanolic solution.

12. A process according to claim 11 comprising the additional step of converting said crystalline solid from step (e) to a salt of an acid other than mandelic acid.

13. A process for preparing a compound of formula 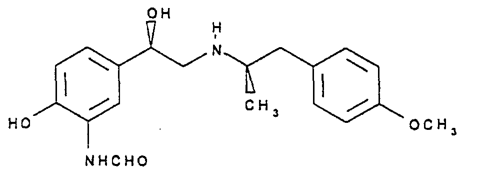 comprising the sequential steps of:
(a) reducing 2-bromo-4'-benzyloxy-3'-nitroacetophenone with about one equivalent of borane-methyl sulfide in the presence of a catalytic amount of a single enantiomer of an oxazaborolidine derived from *cis* 1-amino-2-indanol to produce substantially enantiomerically pure α-(bromomethyl)-4-phenylmethoxy-3-nitrobenzenemethanol; 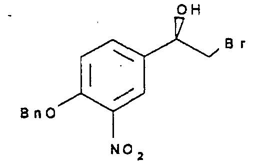
(b) reducing said α-(bromomethyl)-4-phenylmethoxy-3-nitrobenzenemethanol with a source of hydrogen in the presence of a noble metal catalyst to produce an aniline;
(c) formylating said aniline with formic acid and acetic anhydride to produce a compound of formula 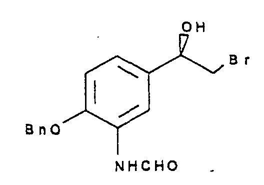
(d) combining said compound of formula 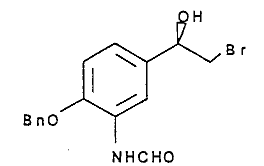 a compound of formula 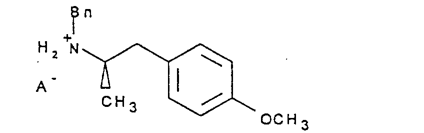 wherein A⁻ is an acid counter ion, and at least one equivalent of a base to produce a mixture comprising an epoxide and a free base;
(e) heating said mixture of an epoxide and a free base at a temperature sufficient to cause a reaction to produce a benzyl-protected aminoalcohol; and
(f) reducing said benzyl-protected aminoalcohol with hydrogen gas in the presence of a noble metal catalyst.

14. A compound of formula: 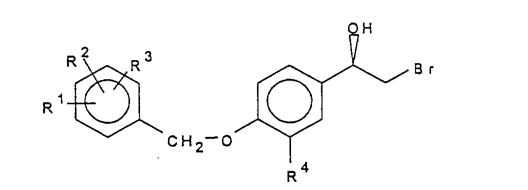 wherein each of R¹, R² and R³ is independently chosen from the group consisting of hydrogen, C₁ to C₆-alkyl, C₁ to C₆-alkoxyl, and halogen and R⁴ is -NO₂, -NH₂ or -NHCHO.

15. A compound according to claim 14 wherein all of R¹, R² and R³ are hydrogen.

16. Crystalline R,R-formoterol L-(+)-tartrate.

17. Solid R,R-formoterol L-(+)-tartrate having a melting point greater than 179°C.

## Patentansprüche

1. Verfahren zur Herstellung einer Verbindung der Formel 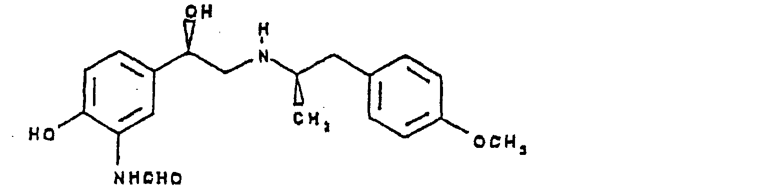 oder eines Salzes hiervon, umfassend die aufeinanderfolgenden Schritte:
(a) Reaktion einer Verbindung der Formel 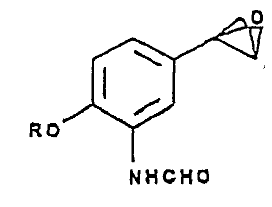 mit einer Verbindung der Formel 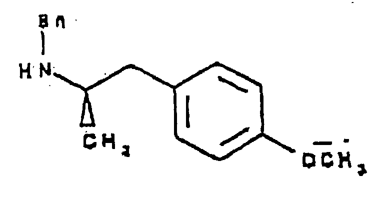 wobei R ein Benzyl oder substituiertes Benzyl ist, und
(b) Reduktion mit einer Wasserstoffquelle in der Gegenwart eines Edelmetall-Katalysators.

2. Verfahren nach Anspruch 1, wobei die Verbindung der Formel 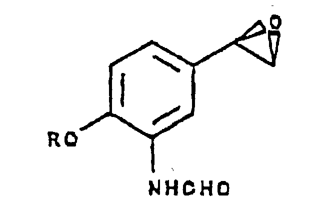 *in situ* aus dem korrespondierenden Bromhydrin hergestellt wird 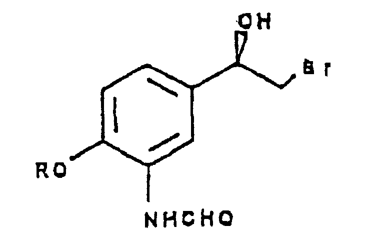 durch Behandlung mit einer Base, und/oder wobei die Verbindung der Formel 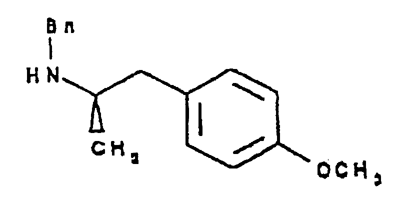 *in situ* hergestellt wird aus dem korrespondieren Salz durch Behandlung mit einer Base.

3. Verfahren nach Anspruch 1 oder 2, wobei die Verbindung der Formel 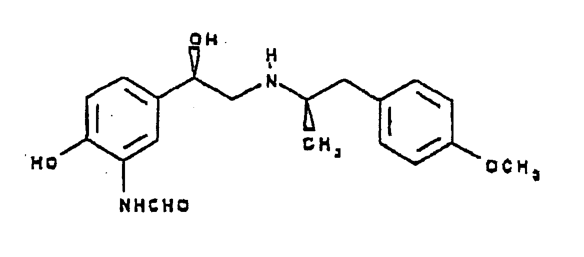
(a) die R,R-Konfiguration oder (b) die S,S-Konfiguration hat.

4. Verfahren zur Herstellung einer Verbindung nach Formel 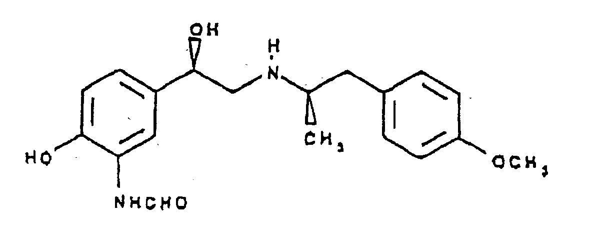 oder eines Salzes hiervon, umfassend die aufeinanderfolgenden Schritte:
(a) Zusammenbringen einer Verbindung der Formel 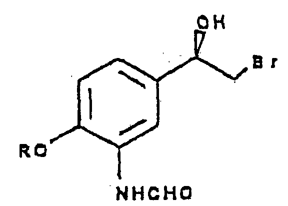 einer Verbindung der Formel 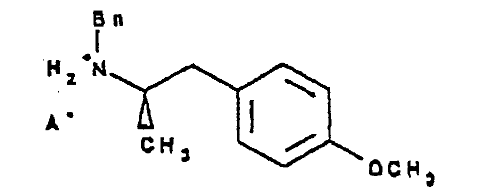 wobei R ein Benzyl oder substituiertes Benzyl und A⁻ ein Säure-Gegenion ist, und mindestens 1 Äquivalents einer Base zur Produktion einer Mischung, umfassend ein Epoxid und eine freie Base;
(b) Erhitzen dieser Mischung eines Epoxids und einer freien Base bei einer Temperatur, die ausreichend ist, um eine Reaktion zur Produktion eines Benzyl-geschützten Aminoalkohols auszulösen; und
(c) Reduktion des Benzyl-geschützten Aminoalkohols mit Wasserstoffgas in Gegenwart eines Edelmetall-Katalysators.

5. Verfahren nach einem der vorhergehenden Ansprüche, wobei der Edelmetall-Katalysator Palladium ist.

6. Verfahren nach Anspruch 2 oder 4, wobei die Base ein Alkalimetall-Carbonat ist.

7. Verfahren nach einem der Ansprüche 1 bis 4, wobei das Salz ein Tartrat-Salz ist.

8. Verfahren nach einem der Ansprüche 1 bis 4, wobei das Salz ein Fumarat-Salz ist.

9. Verfahren zur Synthese einer Verbindung der Formel 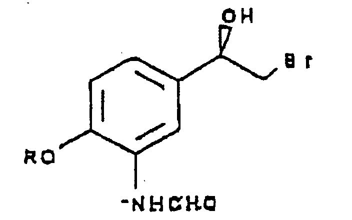 wobei R ein Benzyl oder substituiertes Benzyl ist, umfassend die aufeinanderfolgenden Schritte:
(a) Reduktion von 2-Bromo-4'-RO-3'-nitroacetophenon mit ungefähr einem Äquivalent von Boranmethylsulfid in Gegenwart einer katalytischen Menge einer enantiomeren Form eines Oxazaborolidinreagenz zur Produktion von im wesentlichen enantiomerreinem α-(Bromomethyl)-4-RO-3-nitrobenzolmethanols
(b) Reduktion von α-(Bromomethyl)-4-RO-3-nitrobenzolmethanol mit Wasserstoff in Gegenwart eines Edelmetall-Katalysators zur Produktion eines Anilins; und
(c) Formylieren des Anilins mit Ameisensäure und Essigsäureanhydrid.

10. Verfahren nach Anspruch 9, wobei (a) das Oxazaborolidinreagenz *in situ* aus (1R, 2S)-1-amino-2-indanol und zwei Äquivalenten von Boranmethylsulfid erzeugt wird; und/oder (b) der Edelmetall-Katalysator Platin ist und das Oxazaborolidin aus cis 1-Amino-2-indanol abgeleitet ist; und/oder (c) die Schritte (b) und (c) ohne Isolierung von Anilin ausgeführt werden; und/oder (d) die eine enantiomere Form eines Oxazaborolidins abgeleitet ist aus (1R,2S)-1-Amino-2-indanol, und das im wesentlichen enantiomerreine α-(Bromomethyl)-4-RO-3-nitrobenzolmethanol R-α-(Bromomethyl)-4-phenylmethoxy-3-nitrobenzolmethanol ist.

11. Verfahren zur Herstellung eines im wesentlichen enantiomerreinen Salzes von 4-Methoxy-α-methyl-N-(phenyhnethyl)benzolethanamin, umfassend:
(a) Reduktion von 4-Methoxyphenylaceton mit Wasserstoff in Gegenwart eines Platin-Katalysators und eines Äquivalents von Benzylamin in Methanol;
(b) Hinzufügen eines Äquivalents einer enantiomeren Form von Mandelsäure;
(c) Erhitzen zum Erhalt einer methanolischen Lösung;
(d) Kühlen zum Erhalt einer kristallinen Festphase; und
(e) Entnahme des kristallinen Feststoffes aus der methanolischen Lösung.

12. Verfahren nach Anspruch 11, umfassend den weiteren Schritt einer Umsetzung des kristallinen Feststoffes aus Schritt (e) zu einem Salz einer Säure, die nicht Mandelsäure ist.

13. Verfahren zur Herstellung einer Verbindung der Formel 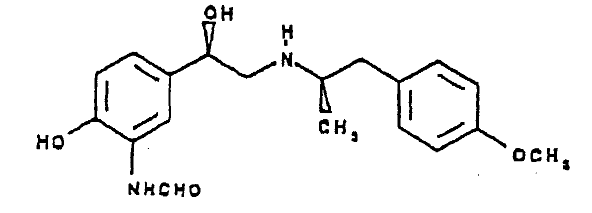 umfassend die aufeinanderfolgenden Schritte:
(a) Reduktion von 2-Bromo-4'-benzyloxy-3'-nitroacetophenon mit ungefähr einem Äquivalent von Boranmethylsulfid in Gegenwart einer katalytischen Menge einer enantiomeren Form eines Oxazaborolidins, abgeleitet aus cis 1-Amino-2-indanol zur Produktion von im wesentlichen enantiomerreinem α-(Bromomethyl)-4-phenylmethoxy-3-nitrobenzolmethanol; 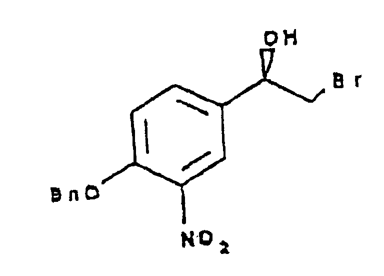
(b) Reduktion von α-(Bromomethyl)-4-phenylmethoxy-3-nitrobenzolmethanol mit einer Wasserstoffquelle in Gegenwart eines Edelmetall-Katalysators zur Produktion eines Anilins;
(c) Formylierung des Anilins mit Ameisensäure und Essigsäureanhydrid zur Herstellung einer Verbindung der Formel 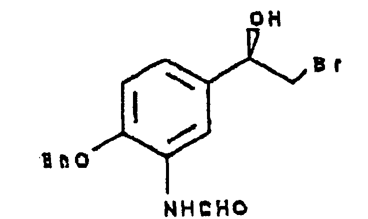
(d) Zusammenbringen der Verbindung der Formel 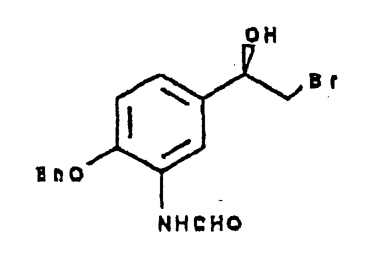 der Verbindung der Formel 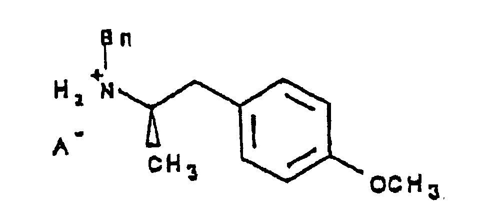 wobei A⁻ ein Säure-Gegenion ist, und mindestens 1 Äquivalents einer Base zur Produktion einer Mischung, umfassend ein Epoxid und eine freie Base;
(e) Erhitzen der Mischung eines Epoxids und einer freien Base bei einer Temperatur, die ausreichend ist, um eine Reaktion zur Produktion eines Benzyl-geschützten Aminoalkohols auszulösen; und
(f) Reduktion des Benzyl-geschützten Aminoalkohols mit Wasserstoffgas in Gegenwart eines Edelmetall-Katalysators.

14. Verbindung der Formel 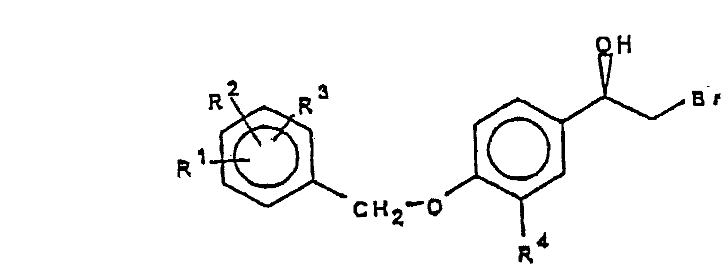 wobei R¹, R² und R³ jeweils unabhängig gewählt sind aus einer Gruppe, bestehend aus Wasserstoff, C₁- bis C₆-Alkyl, C₁- bis C₆-Alkoxyl, und Halogen, und R⁴ -NO₂, NH₂ oder NHCHO ist.

15. Verbindung nach Anspruch 14, wobei R¹, R² und R³ jeweils Wasserstoff sind.

16. Kristallines R,R-Formoterol-L-(+)-Tartrat.

17. Festes R,R-Formoterol-L-(+)-Tartrat mit einem Schmelzpunkt größer als 179°C.

## Revendications

1. Procédé pour préparer un composé ayant la formule suivante : 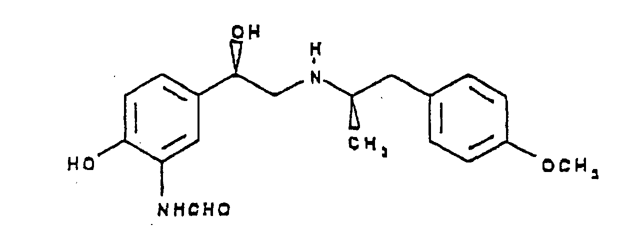 ou un sel de celui-ci, comportant les étapes séquentielles consistant à :
(a) faire réagir un composé ayant la formule suivante : 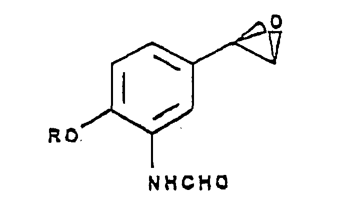 avec un composé ayant la formule suivante 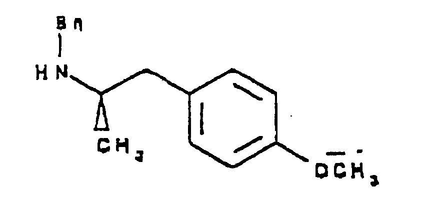 où R est un benzyle ou un benzyle substitué, et
(b) réduire à l'aide d'une source d'hydrogène en présence d'un catalyseur de métal noble.

2. Procédé selon la revendication 1, dans lequel ledit composé ayant la formule suivante : 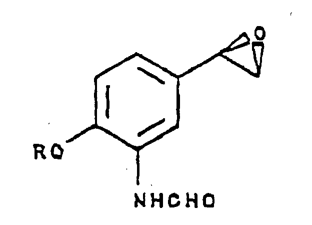 où R est un benzyle ou un benzyle substitué, est produit *in situ* à partir de la bromohydrine correspondante 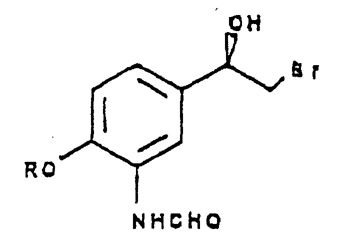 par traitement à l'aide d'une base, et/ou dans lequel ledit composé ayant la formule suivante 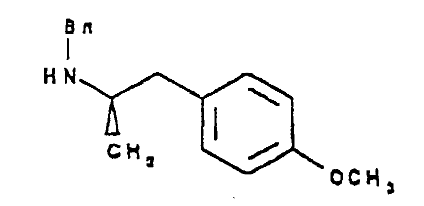 où B₁₁ est un benzyle, est produit *in situ* à partir d'un sel correspondant par traitement à l'aide d'une base.

3. Procédé selon la revendication 1 ou 2, dans lequel ledit composé ayant la formule suivante 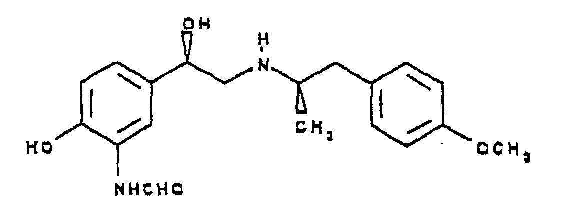 est : (a) de la configuration R, R, ou (b) de la configuration S, S.

4. Procédé pour préparer un composé ayant la formule suivante : 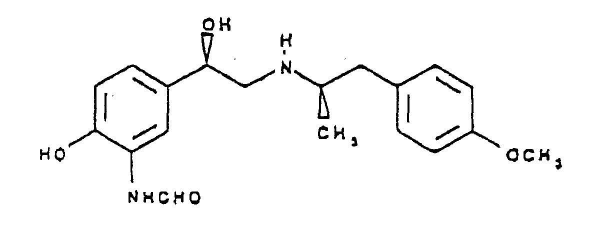 où un sel de celui-ci, comportant les étapes séquentielles consistant à :
(a) combiner un composé ayant la formule suivante 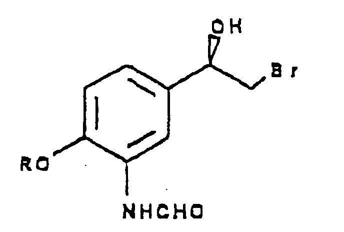 et un composé ayant la formule suivante 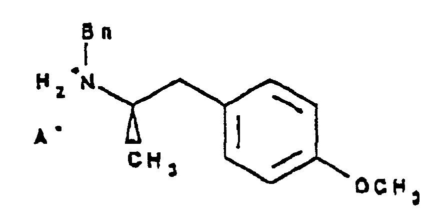 où R est un benzyle ou un benzyle substitué et A⁻ est un contre-ion acide, et au moins un équivalent d'une base pour produire un mélange comportant un époxyde et une base libre,
(b) chauffer ledit mélange constitué d'un époxyde et d'une base libre à une température suffisante pour amener une réaction à produire un aminoalcool protégé par un groupe benzyle, et
(c) réduire ledit aminoalcool protégé par un groupe benzyle à l'aide d'hydrogène gazeux en présence d'un catalyseur de métal noble.

5. Procédé selon l'une quelconque des revendications précédentes, dans lequel ledit catalyseur de métal noble est du palladium.

6. Procédé selon la revendication 2 ou 4, dans lequel ladite base est un carbonate de métal alcalin.

7. Procédé selon l'une quelconque des revendications 1 à 4, dans lequel ledit sel est un sel de tartrate.

8. Procédé selon l'une quelconque des revendications 1 à 4, dans lequel ledit sel est un sel de fumarate.

9. Procédé pour synthétiser un composé ayant la formule suivante : 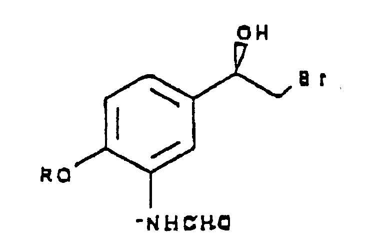 où R est un benzyle ou un benzyle substitué, comportant les étapes séquentielles consistant à :
(a) réduire le composé 2-bromo-4'-RO-3'-nitroacétophénone avec environ un équivalent de sulfure de borane-méthyle en présence d'une quantité catalytique d'un énantiomère unique d'un réactif d'oxazaborolidine pour produire un composé α-(bromométhyle)-4-RO-3-nitrobenzèneméthanol pratiquement pur d'un point de vue énantiomère,
(b) réduire ledit composé α-(bromométhyle)-4-RO-3-nitrobenzèneméthanol à l'aide d'hydrogène en présence d'un catalyseur de métal noble pour produire une aniline, et
(c) formyler ladite aniline à l'aide d'acide formique et d'anhydride acétique.

10. Procédé selon la revendication 9, dans lequel : (a) ledit réactif d'oxazaborolidine est généré *in situ* à partir de (1R,2S)-1-amino-2-indanol et deux équivalents de sulfure de borane-méthyle, et/ou (b) ledit catalyseur de métal noble est du platine et ledit oxazaborolidine est dérivé de *cis* 1-amino-2-indanol, et/ou (c) les étapes (b) et (c) sont effectuées sans isolation de ladite aniline, et/ou (d) ledit énantiomère unique d'un oxazaborolidine est dérivé de (1R,2S)-1-amino-2-indanol, et ledit composé α-(bromométhyle)-4-RO-3-nitrobenzèneméthanol pratiquement pur d'un point de vue énantiomère est le composé R-α-(bromométhyle)-4-phénylméthoxy-3-nitrobenzèneméthanol.

11. Procédé pour préparer un sel pratiquement pur d'un point de vue énantiomère de 4-méthoxy-α-méthyle-N-(phénylméthyle)benzèneéthanamine comportant les étapes consistant à :
(a) réduire de l'acétone 4-méthoxyphénylique à l'aide d'hydrogène en présence d'un catalyseur de platine et environ 1 équivalent de benzylamine dans du méthanol,
(b) ajouter un équivalent d'un énantiomère unique d'acide mandélique,
(c) chauffer pour obtenir une solution méthanolique,
(d) refroidir pour obtenir une phase solide cristalline, et
(e) récupérer ladite phase solide cristalline à partir de ladite solution méthanolique.

12. Procédé selon la revendication 11, comportant l'étape supplémentaire consistant à convertir ladite phase solide cristalline provenant de l'étape (e) en un sel d'un acide autre que l'acide mandélique.

13. Procédé pour préparer un composé ayant la forme suivante : 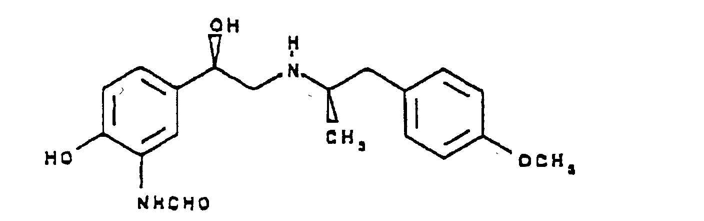 comportant les étapes séquentielles consistant à :
(a) réduire le composé 2-bromo-4'-benzyloxy-3'-nitroacétophénone à l'aide d'environ un équivalent de sulfure de borane-méthyle en présence d'une quantité catalytique d'un énantiomère unique d'un oxazaborolidine dérivé de *cis* 1-amino-2-indanol pour produire le composé α-(bromométhyle)-4-phénylméthoxy-3-nitrobenzèneméthanol pratiquement pur d'un point de vue énantiomère : 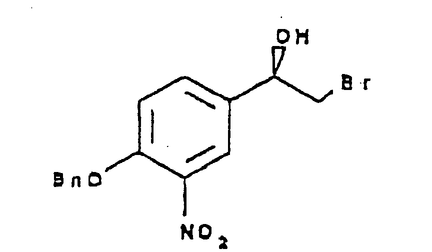
(b) réduire ledit composé α-(bromométhyle)-4-phénylméthoxy-3-nitrobenzèneméthanol à l'aide d'une source d'hydrogène en présence d'un catalyseur de métal noble pour produire une aniline,
(c) formyler ladite aniline à l'aide d'acide formique et d'anhydride acétique pour produire un composé ayant la formule suivante :
(d) combiner ledit composé ayant la formule suivante 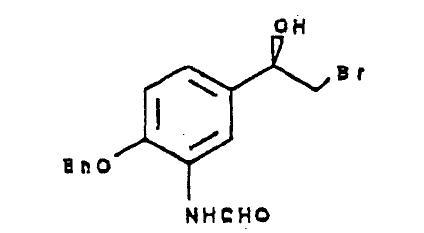 et un composé ayant la formule suivante 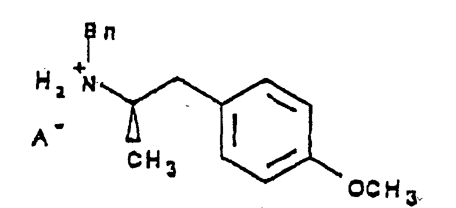 où A⁻ est un contre-ion acide, et au moins un équivalent d'une base pour produire un mélange comportant un époxyde et une base libre,
(e) chauffer ledit mélange constitué d'un époxyde et d'une base libre à une température suffisante pour amener une réaction à produire un aminoalcool protégé par un groupe benzyle, et
(f) réduire ledit aminoalcool protégé par un groupe benzyle à l'aide d'hydrogène gazeux en présence d'un catalyseur de métal noble.

14. Composé ayant la formule suivante : 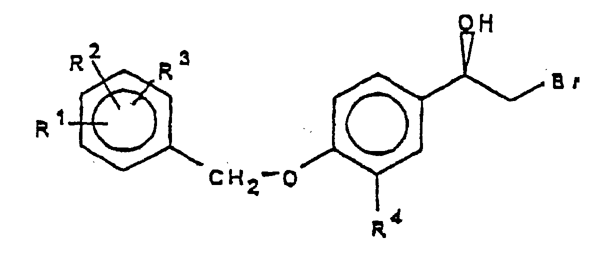 où R¹, R² et R³ sont chacun indépendamment choisis parmi le groupe constitué d'hydrogène, d'alkyle C₁ à C₆, d'alkoxyle C₁ à C₆, et d'halogène et R⁴ est -NO₂, -NH₂, ou -NHCHO.

15. Composé selon la revendication 14, dans lequel R¹, R² et R³ sont tous de l'hydrogène.

16. R,R-formotérol-L-(+)-tartrate cristallin.

17. R,R-formotérol-L-(+)-tartrate solide ayant un point de fusion supérieur à 179°C.
